(19) **Europäisches Patentamt**
**European Patent Office**
**Office européen des brevets**

(11) **EP 4 729 047 A1**

(12) **EUROPEAN PATENT APPLICATION**
published in accordance with Art. 153(4) EPC

(43) Date of publication:
**22.04.2026 Bulletin 2026/17**

(21) Application number: **24823749.7**

(22) Date of filing: **14.06.2024**

(51) International Patent Classification (IPC):
**A61K 8/85** *(2006.01)*  **A61K 8/02** *(2006.01)*
**A61K 8/29** *(2006.01)*  **A61Q 19/00** *(2006.01)*
**A61Q 17/04** *(2006.01)*

(52) Cooperative Patent Classification (CPC):
**A61K 8/02; A61K 8/29; A61K 8/85; A61Q 17/04;**
**A61Q 19/00**

(86) International application number:
**PCT/KR2024/008235**

(87) International publication number:
**WO 2024/258233 (19.12.2024 Gazette 2024/51)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **16.06.2023 KR 20230077464**

(71) Applicant: **CJ Cheiljedang Corporation**
**Seoul 04560 (KR)**

(72) Inventors:
• **MUN, Bo Ra**
  **Seoul 04560 (KR)**
• **LEE, Eun-Hye**
  **Seoul 04560 (KR)**
• **YOON, Ki Chull**
  **Seoul 04560 (KR)**

(74) Representative: **Meissner Bolte Partnerschaft**
**mbB**
**Patentanwälte Rechtsanwälte**
**Postfach 86 06 24**
**81633 München (DE)**

(54) **COSMETIC COMPOSITION COMPRISING BIODEGRADABLE POLYMER PARTICLES**

(57) The present application relates to a cosmetic composition comprising a biodegradable polymer resin containing a PHA resin and having a particle diameter adjusted to a specific range. The cosmetic composition of the present application can be used in the manufacture of various products, such as basic cosmetics, color cosmetics, sunscreen cosmetics, and hair care cosmetics.

## Description

### Technical Field

The present disclosure relates to a cosmetic composition comprising biodegradable polymer particles. More specifically, the present disclosure relates to a cosmetic composition using biodegradable polymer particles comprising a polyhydroxyalkanoate (PHA).

### Background Art

Microplastics, the main cause of environmental pollution, are the biggest issue in the cosmetics industry. There is a worldwide ban on the distribution of cosmetics that contain solid plastic particles (microplastics) of 5 mm or less used for the purposes of cleansing, exfoliation, and the like, as long as wash-off products are concerned. There is also a grace period for general products (basic cosmetics, sunscreens, makeup products) that are not washed off, and the ban on their use will come into effect soon.

In the case of sunscreens, if the user goes into the ocean once it has been applied, harmful ingredients may be washed away into the water, causing serious damage to coral and fish and having a significant adverse impact on the marine ecosystem. The banned ingredients are octinoxate (ethylhexyl methoxycinnamate) and oxybenzone (benzophenone-3), which are known to cause coral bleaching and damage the coral's DNA. In addition, they are known to be highly toxic ingredients that cause allergies and affect the respiratory and digestive systems; thus, they are rated as dangerous by the Environmental Working Group (EWG), a non-profit organization in the United States. Coral reefs, which play a key role in the ecosystem, are essential for the survival of a large number of marine species. In addition, since the ocean covers 70% of the Earth's surface, protecting the marine ecosystem is very crucial. To protect the marine ecosystem, banned ingredients have been designated for sunscreens, and regulations on the use of microplastics are being strengthened.

As discussed above, the cosmetics industry is increasingly considering the environment and using safe raw materials to protect health.

[Prior Art Document]

(Patent document 1) Japanese Laid-open Patent Publication No. 2015-522065

### Detailed Description of the Invention

### Technical Problem

Polyhydroxyalkanoate (PHA), as a biodegradable polymer, is biocompatible, non-cytotoxic, and has the effect of promoting the regeneration of human dermal fibroblasts, which can help restore damaged skin. Thus, since it is safe when used on the skin and produces various effects, it can be applied widely to personal care products such as base and color cosmetics, sunscreens, and hair care cosmetics.

As a result of research on cosmetic compositions using polyhydroxyalkanoate (PHA) particles conducted by the present inventors, it has been discovered that a cosmetic composition comprising a biodegradable polymer resin comprising a copolymeric PHA resin and having a particle diameter controlled to a specific range is excellent in terms of usability, sensory properties, UV blocking capability, hiding power, non-toxicity, and formulation stability.

Accordingly, an object of the present disclosure is to provide a cosmetic composition that has excellent characteristics as a cosmetic while being biodegradable and not polluting the environment.

### Solution to the Problem

The present disclosure provides a cosmetic composition, which comprises biodegradable polymer particles, wherein the biodegradable polymer particles comprise a copolymeric polyhydroxyalkanoate (PHA), the copolymeric polyhydroxyalkanoate (PHA) comprises a repeat unit derived from 4-hydroxybutyrate (4-HB) in an amount of 1% by weight to 20% by weight based on the total weight of the copolymeric polyhydroxyalkanoate (PHA), and the biodegradable polymer particles have an average particle diameter of 20 μm or less.

In one embodiment, wherein the copolymeric polyhydroxyalkanoate (PHA) further comprises a repeat unit derived from at least one monomer selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-Hhep), 3-hydroxyoctanoate (3-HO), 3-hydroxynonanoate (3-HN), 3-hydroxydecanoate (3-HD), 3-hydroxydodecanoate (3-HDd), 4-hydroxyvalerate (4-HV), 5-hydroxyvalerate (5-HV), and 6-hydroxyhexanoate (6-HH).

In another embodiment, wherein the copolymeric polyhydroxyalkanoate (PHA) comprises a poly(3-hydroxybutyrate-co-4-hydroxybutyrate) (P3HB-co-4HB).

In yet another embodiment, wherein the copolymeric polyhydroxyalkanoate (PHA) comprises a repeat unit derived from 4-hydroxybutyrate (4-HB) in an amount of 3% by weight to 15% by weight based on the total weight of the copolymeric polyhydroxyalkanoate (PHA).

In yet another embodiment, wherein the copolymeric polyhydroxyalkanoate (PHA) is employed in an amount of 0.1% by weight to 30% by weight based on the total weight of the cosmetic composition.

In yet another embodiment, wherein the cosmetic composition further comprises at least one selected from the group consisting of a UV blocking agent, an oil, a surfactant, and a polyol.

In yet another embodiment, wherein the UV blocking agent is at least one selected from the group consisting of titanium dioxide ($TiO_2$), zinc oxide (ZnO), drometrizole, drometrizole trisiloxane, digalloyl trioleate, disodium phenyl dibenzimidazole tetrasulfonate, diethylamino hydroxybenzoyl hexyl benzoate, diethylhexyl butamido triazone, methylene bis-benzotriazolyl tetramethylbutylphenol, methyl anthranilate, benzophenone-4, benzophenone-8, bis-ethylhexyloxyphenol methoxyphenyl triazine, ethylhexyl dimethyl PABA, ethylhexyl salicylate, ethylhexyl triazone, isoamyl p-methoxycinnamate, terephthalylidene dicamphor sulfonic acid, phenylbenzimidazole sulfonic acid, and polysilicone-15.

In yet another embodiment, wherein the oil is at least one selected from the group consisting of natural oils, vegetable oils, waxes, hydrocarbons, higher fatty acids, higher alcohols, and silicone oils.

In yet another embodiment, wherein the surfactant is at least one selected from the group consisting of anionic surfactants, cationic surfactants, amphoteric surfactants, nonionic surfactants, polymeric surfactants, natural surfactants, and silicone surfactants.

In yet another embodiment, wherein the content of the surfactant is 0.1% by weight to 30% by weight based on the total weight of the cosmetic composition.

In yet another embodiment, wherein the content of the UV blocking agent is 5% by weight to 35% by weight based on the total weight of the cosmetic composition.

In yet another embodiment, wherein the content of the oil is 0.1% by weight to 70% by weight based on the total weight of the cosmetic composition.

In yet another embodiment, wherein the cosmetic composition has an SPF (sun protection factor) of 30 or more and a PA (protection factor of UVA) of 8 or more.

In yet another embodiment, wherein the cosmetic composition has the formulation of a sun stick, sun lotion, sunscreen, sun balm, sun spray, softening toner, nourishing toner, lotion, nourishing cream, massage cream, essence, ampoule, eye cream, makeup base cream, primer, foundation, pact, shampoo, treatment, scalp ampoule, pack, spray, body wash, cleansing foam, cleansing lotion, cleansing oil, or powder.

In yet another embodiment, wherein the cosmetic composition is used in the manufacture of basic cosmetics, color cosmetics, sunscreen cosmetics, or hair care cosmetics.

The present disclosure also provides a cosmetic composition for UV blocking, which comprises biodegradable polymer particles, wherein the biodegradable polymer particles comprise a copolymeric polyhydroxyalkanoate (PHA), the copolymeric polyhydroxyalkanoate (PHA) comprises a repeat unit derived from 4-hydroxybutyrate (4-HB) in an amount of 1% by weight to 20% by weight based on the total weight of the copolymeric polyhydroxyalkanoate (PHA), and the cosmetic composition has an SPF (sun protection factor) of 30 or more and a PA (protection factor of UVA) of 8 or more.

The present disclosure also provides a cosmetic composition for skin regeneration, which comprises biodegradable polymer particles, wherein the biodegradable polymer particles comprise a copolymeric polyhydroxyalkanoate (PHA), and the copolymeric polyhydroxyalkanoate (PHA) comprises a repeat unit derived from 4-hydroxybutyrate (4-HB) in an amount of 1% by weight to 20% by weight based on the total weight of the copolymeric polyhydroxyalkanoate (PHA).

**Advantageous Effects of the Invention**

The cosmetic composition according to the present disclosure, which comprises a biodegradable polymer resin comprising a copolymeric PHA resin and having a particle diameter controlled to a specific range, is excellent in terms of usability, sensory properties, UV blocking capability, hiding power, non-toxicity, and formulation stability.

Specifically, the cosmetic composition has excellent UVB and UVA blocking booster effects, which can reduce the amount of UV filters that cause irritation in the formulation and help protect the skin from damage caused by ultraviolet rays. In addition, because the PHA particles are round, they provide a smooth feeling of use and improve spreadability, and they impart a soft focusing effect to the skin, thereby helping smoothen the skin texture. In addition, since PHA has a high melting point, it remains stable without changing its particle or shape even when processed at low or high temperatures. Thus, there is no problem when it is applied to emulsified formulations such as lotions and creams that require a process operated at high temperatures.

In particular, the cosmetic composition can be applied to various cosmetics such as cosmetics using mineral or organic UV filters or basic and color cosmetics. Further, since it can be easily biodegraded in both soil and the ocean, it can effectively contribute to environmental conservation.

As described above, since the cosmetic composition of the present disclosure is excellent in terms of the feeling of use, UV blocking booster effect, cell proliferation rate, and the like, it can be used in the manufacture of various products such as basic cosmetics, color cosmetics, sunscreen cosmetics, and hair care cosmetics.

## Best Mode for Carrying out the Invention

Hereinafter, the present disclosure will be described in detail with reference to the embodiments. The embodiments are not limited to those described below. Rather, they can be modified into various forms as long as the gist of the invention is not altered.

In the present specification, the term "comprising" is intended to specify a particular characteristic, region, step, process, element, and/or component. It does not exclude the presence or addition of any other characteristic, region, step, process, element and/or component, unless specifically stated to the contrary.

In addition, all numbers expressing the physical properties, dimensions, and the like of elements used herein are to be understood as being modified by the term "about" unless otherwise indicated.

In the numerical range that limits the size of components, physical properties, and the like described in the present specification, when a numerical range limited with the upper limit only and a numerical range limited with the lower limit only are separately exemplified, it should be understood that a numerical range combining these upper and lower limits is also encompassed in the exemplary scope.

## Biodegradable polymer particles

The cosmetic composition according to the present disclosure comprises biodegradable polymer particles.

Specifically, the biodegradable polymer particles comprise a polyhydroxyalkanoate (PHA).

The polyhydroxyalkanoate (PHA) may have physical properties similar to those of conventional petroleum-derived synthetic polymers such as polybutylene adipate terephthalate (PBAT), polybutylene succinate (PBS), polybutylene succinate terephthalate (PBST), and polybutylene succinate adipate (PBSA), exhibits excellent biodegradability, and is excellent in biocompatibility.

Specifically, PHA, which is a natural polyester polymer that accumulates within microbial cells, can be completely decomposed into carbon dioxide, water, organic waste, and the like in soil and/or the ocean; thus, it can have excellent biodegradability without generating microplastics. Accordingly, the cosmetic composition according to the present disclosure can be excellent in biodegradability and environmental friendliness as it comprises such a PHA.

In particular, the PHA particles themselves are round in shape and processed to be small in particle size. When applied to base or color cosmetics, they can provide a soft focusing effect and help improve the feeling of use.

In addition, since PHA has the function of an SPF booster, when it is made into a sunscreen together with a UV blocking agent ($TiO_2$, ZnO, or the like), it can increase spreadability and exhibit a higher UV protection effect. UVA (long wavelength of 320 to 400 nm) has only 1/1,000 the energy of UVB (short wavelength of 280 to 320 nm), while the amount of light reaching the ground is 100 times that of UVB, and it is known to be the main cause of skin pigmentation and aging. PHA has an excellent UVA blocking booster effect in addition to UVB blocking, and it has the advantage of blocking a wider range of ultraviolet rays than organic absorbents.

The PHA may be obtained by cell disruption using a mechanical method or a physical method, or it may be obtained by cell disruption using a non-mechanical method or a chemical method. Specifically, the PHA may be a copolymer obtained through an enzyme-catalyzed polymerization process of one or more monomers within living microbial cells.

More specifically, the biodegradable polymer particles comprise a copolymeric polyhydroxyalkanoate (PHA).

The copolymeric polyhydroxyalkanoate (PHA) may comprise a copolymer comprising two or more different repeat units with the different repeat units randomly distributed in the polymer chains.

In an embodiment, the copolymeric polyhydroxyalkanoate (PHA) may comprise a repeat unit derived from 4-hydroxybutyrate (4-HB).

As the copolymeric PHA comprises a repeat unit derived from 4-hydroxybutyrate (4-HB), the physical properties and effects of the cosmetic composition can be improved. As a result, it is possible to provide a cosmetic composition that can be used for various purposes.

In the present disclosure, it is important to adjust the content of the 4-HB repeat unit in the copolymeric PHA. That is, in order to achieve the physical properties desired in the present disclosure, in particular, to increase biodegradability *in vivo* and to achieve excellent mechanical properties, the content of the 4-HB repeat unit in the copolymeric PHA may be important.

The content of a repeat unit derived from 4-hydroxybutyrate (4-HB) may be 0.1% by weight or more, 1% by weight or more, 3% by weight or more, 5% by weight or more, 7% by weight or more, 8% by weight or more, 10% by weight or more, 12% by weight or more, 13% by weight or more, 15% by weight or more, 17% by weight or more, 18% by weight or more, 20% by weight or more, or 25% by weight or more, and 50% by weight or less, 45% by weight or less, 43% by weight or less, 42% by weight or less, 40% by weight or less, 35% by weight or less, 30% by weight or less, 25% by weight or less, or 20% by weight or less, based on the total weight of the copolymeric polyhydroxyalkanoate (PHA).

In an embodiment, the copolymeric polyhydroxyalkanoate (PHA) may comprise a repeat unit derived from 4-hydroxybutyrate (4-HB) in an amount of 1% by weight to 20% by weight based on the total weight of the copolymeric polyhydroxyalkanoate (PHA).

For example, the content of a repeat unit derived from 4-hydroxybutyrate (4-HB) may be 1% by weight to 20% by weight, 2% by weight to 20% by weight, 3% by weight to 15% by weight, 5% by weight to 15% by weight, or 5% by weight to 10% by weight, based on the total weight of the copolymeric polyhydroxyalkanoate (PHA), but it is not limited thereto.

As a specific example, the copolymeric polyhydroxyalkanoate (PHA) may comprise a repeat unit derived from 4-hydroxybutyrate (4-HB) in an amount of 3% by weight to 15% by weight based on the total weight of the copolymeric polyhydroxyalkanoate (PHA).

When the content of the 4-HB repeat unit in the copolymeric polyhydroxyalkanoate (PHA) satisfies the above range, the biodegradability in soil and the ocean can be enhanced, and the stability and physical properties as a cosmetic raw material can be maintained to be excellent.

As described above, the copolymeric PHA comprises one or more of a 4-HB repeat unit, and the content of the 4-HB repeat unit may be controlled to adjust the crystallinity of the copolymeric PHA. That is, the copolymeric PHA may be a polymer with controlled crystallinity.

The copolymeric PHA whose crystallinity has been adjusted may be one in which its crystallinity and amorphousness are adjusted as the irregularities are increased in its molecular structure. Specifically, the types and ratios of the monomers or the types and/or contents of the isomers may be adjusted.

In addition, the copolymeric PHA may be a PHA copolymer that comprises a 4-HB repeat unit and further comprises one repeat unit different from the 4-HB repeat unit, or two, three, four, five, six, or more repeat units different from each other.

For example,, the copolymeric polyhydroxyalkanoate (PHA) may further comprise a repeat unit derived from at least one monomer selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-Hhep), 3-hydroxyoctanoate (3-HO), 3-hydroxynonanoate (3-HN), 3-hydroxydecanoate (3-HD), 3-hydroxydodecanoate (3-HDd), 4-hydroxyvalerate (4-HV), 5-hydroxyvalerate (5-HV), and 6-hydroxyhexanoate (6-HH).

Specifically, the copolymeric polyhydroxyalkanoate (PHA) may comprise at least one repeat unit selected from the group consisting of 3-hydroxybutyrate (3-HB), 3-hydroxypropionate (3-HP), 3-hydroxyvalerate (3-HV), 3-hydroxyhexanoate (3-HH), 4-hydroxyvalerate (4-HV), 5-hydroxyvalerate (5-HV), and 6-hydroxyhexanoate (6-HH).

More specifically, the copolymeric PHA may comprise a copolymer comprising a repeat unit derived from 3-HB and a repeat unit derived from 4-HB. For example, the copolymeric PHA may comprise a poly(3-hydroxybutyrate-co-4-hydroxybutyrate) (3HB-co-4HB).

For example, the content of a repeat unit derived from the 3-HB may be 50% by weight or more, 55% by weight or more, 60% by weight or more, 64% by weight or more, 70% by weight or more, 75% by weight or more, or 80% by weight or more, and 99.9% by weight or less, 99% by weight or less, 95% by weight or less, 90% by weight or less, 85% by weight or less, 80% by weight or less, or 75% by weight or less, based on the total weight of the copolymeric PHA.

In addition, the copolymeric PHA may comprise isomers. For example, the copolymeric PHA may comprise structural isomers, enantiomers, or geometric isomers. Specifically, the PHA may comprise structural isomers.

The copolymeric PHA may have a glass transition temperature (Tg) of, for example, -45°C to 80°C, -35°C to 80°C, -30°C to 80°C, -25°C to 75°C, -20°C to 70°C, - 35°C to 5°C, -25°C to 5°C, -35°C to 0°C, -25°C to 0°C, -30°C to -10°C, -35°C to -15°C, -35°C to -20°C, -20°C to 0°C, -15°C to 0°C, or -15°C to -5°C.

The crystallization temperature (Tc) of the copolymeric PHA, for example, may not be measured or may be, for example, 70°C to 120°C, 75°C to 120°C, 75°C to 115°C, 75°C to 110°C, or 90°C to 110°C.

The melting temperature (Tm) of the copolymeric PHA, for example, may not be measured or may be, for example, 100°C to 170°C, 110°C to 150°C, or 120°C to 140°C.

The copolymeric PHA may have a weight average molecular weight (Mw) of, for example, 10,000 g/mole to 1,200,000 g/mole. For example, the weight average molecular weight of the copolymeric PHA may be 50,000 g/mole to 1,200,000 g/mole, 100,000 g/mole to 1,200,000 g/mole, 50,000 g/mole to 1,000,000 g/mole, 100,000 g/mole to 1,000,000 g/mole, 100,000 g/mole to 900,000 g/mole, 200,000 g/mole to 1,200,000 g/mole, 250,000 g/mole to 1,150,000 g/mole, 300,000 g/mole to 1,100,000 g/mole, 350,000 g/mole to 1,000,000 g/mole, 350,000 g/mole to 950,000 g/mole, 100,000 g/mole to 900,000 g/mole, 200,000 g/mole to 800,000 g/mole, 200,000 g/mole to 700,000 g/mole, 250,000 g/mole to 650,000 g/mole, 200,000 g/mole to 400,000 g/mole, 300,000 g/mole to 800,000 g/mole, 300,000 g/mole to 600,000 g/mole, 400,000 g/mole to 800,000 g/mole, 500,000 g/mole to 1,200,000 g/mole, 500,000 g/mole to 1,000,000 g/mole 550,000 g/mole to 1,050,000 g/mole, 550,000 g/mole to 900,000 g/mole, or 600,000 g/mole to 900,000 g/mole.

In an embodiment, the copolymeric PHA may be a crystalline or semi-crystalline PHA. For example, the crystalline or semi-crystalline PHA may comprise a 4-HB repeat unit in an amount of, for example, 1% by weight to 25% by weight or 3% by weight to 20% by weight based on the total weight of the copolymeric PHA. In addition, the crystalline or semi-crystalline PHA may have a glass transition temperature (Tg) of, for example, -20°C to 0°C, a crystallization temperature (Tc) of, for example, 75°C to 115°C, and a melting temperature (Tm) of, for example, 110°C to 160°C.

In another embodiment, the biodegradable polymer particles may further comprise other PHA-based polymers. For example, it may further comprise at least one polymer selected from the group consisting of poly(3-hydroxybutyrate) (P3HB), poly(4-hydroxybutyrate) (P4HB), poly(3-hydroxyhexanoate) (P3HH), poly(3-hydroxybutyrate-co-3-hydroxyhexanoate) (P3HB-3HH), poly(3-hydroxyoctanoate) (P3HO), and poly(3-hydroxybutyrate-co-3-hydroxyoctanoate) (P3HB-3HO).

In another embodiment, the biodegradable polymer particles may be one, or a mixture of two or more, selected from synthetic biodegradable polymers and natural biodegradable polymers other than polyhydroxyalkanoate (PHA). Examples of the synthetic biodegradable polymer include polylactic acid (PLA), polycaprolactone (PCL), polyglycolic acid (PGA), poly(lactic acid-co-caprolactone) (PLCL), poly(lactic acid-co-glycolic acid) (PLGA), polybutylene succinate (PBS), and poly(butylene adipate-co-terephthalate) (PBAT). Examples of the natural biodegradable polymer include starch, silk fibroin, chitosan, chitin, cellulose, collagen, and gelatin.

The particle diameter of the biodegradable polymer particles may be controlled to a specific range.

In an embodiment, the biodegradable polymer particles have an average particle diameter of 20 μm or less.

The average particle diameter may be, for example, the particle diameter corresponding to a cumulative percentage of 50%, i.e., D50, in a particle size distribution.

Within the above particle diameter range, it is suitable for use in basic cosmetics, color cosmetics, and sunscreens. On the other hand, if it is outside the range, the feeling of use may not be good, and there may be a feeling of foreign substances.

Specifically, the average particle diameter of the biodegradable polymer particles may be 1 μm to 10 μm, more specifically, 2 μm to 8 μm.

The biodegradable polymer particles may be ground into small particles using a food mixer or an atomizer to obtain a size suitable for a cosmetic formulation. In addition, to obtain a suitable particle size, the grinding operation may be repeated 2 to 3 times. In addition to the grinding equipment described above, they may also be obtained through various fine particle grinders (jet mill, air jet mill, and the like). In addition, the biodegradable polymer particles may be fed to a circular vibration sorter and sorted to obtain particles with a desired particle size.

**Cosmetic composition**

As described above, the cosmetic composition of the present disclosure is prepared using biodegradable polymer particles comprising a copolymeric polyhydroxyalkanoate (PHA).

The content of the biodegradable polymer particles may be 0.1% by weight or more, 0.5% by weight or more, 1% by weight or more, 2% by weight or more, 3% by weight or more, 5% by weight or more, 7% by weight or more, 8% by weight or more, 10% by weight or more, 15% by weight or more, 20% by weight or more, or 25% by weight or more, and may be 50% by weight or less, 40% by weight or less, 30% by weight or less, 25% by weight or less, 20% by weight or less, or 15% by weight or less, based on the total weight of the cosmetic composition.

Specifically, the content of the biodegradable polymer particles may be 0.1% by weight to 50% by weight, 0.1% by weight to 30% by weight, 1% by weight to 30% by weight, 1% by weight to 25% by weight, 1% by weight to 20% by weight, 1% by weight to 15% by weight, 1.5% by weight to 10% by weight, 2% by weight to 10% by weight, 2.5% by weight to 10% by weight, 3% by weight to 50% by weight, 3% by weight to 30% by weight, 3% by weight to 20% by weight, 3% by weight to 10% by weight, 4% by weight to 10% by weight, or 5% by weight to 10% by weight, based on the total weight of the cosmetic composition.

In addition, the content of the copolymeric polyhydroxyalkanoate (PHA) may be 0.1% by weight or more, 0.2% by weight or more, 0.5% by weight or more, 1% by weight or more, 1.5% by weight or more, 2% by weight or more, 2.5% by weight or more, 3% by weight or more, 5% by weight or more, 10% by weight or more, 15% by weight or more, or 20% by weight or more, and 50% by weight or less, 40% by weight or less, 30% by weight or less, 25% by weight or less, 20% by weight or less, 15% by weight or less, or 10% by weight or less, based on the total weight of the cosmetic composition.

In an embodiment, the copolymeric polyhydroxyalkanoate (PHA) may be employed in an amount of 0.1% by weight to 30% by weight based on the total weight of the cosmetic composition. Specifically, the copolymeric polyhydroxyalkanoate (PHA) may be employed in an amount of 1% by weight to 20% by weight, 1% by weight to 15% by weight, 2% by weight to 10% by weight, 2.5% by weight to 10% by weight, 3% by weight to 30% by weight, 3% by weight to 20% by weight, 3% by weight to 10% by weight, or 5% by weight to 10% by weight, based on the total weight of the cosmetic composition.

Within the above preferred range, biodegradability and cell proliferation effects can be further improved, and other cosmetic properties can also be more advantageous.

The cosmetic composition may further comprise at least one selected from the group consisting of a UV blocking agent, an oil, a surfactant, and a polyol.

The cosmetic composition according to the present disclosure may comprise a UV blocking agent. The UV blocking agent serves to block UVA (long wavelength of 320 to 400 nm) and UVB (short wavelength of 280 to 320 nm) rays from reaching the skin, thereby preventing pigmentation and aging.

The UV blocking agent may be, for example, at least one selected from the group consisting of a mineral UV blocking agent, an organic UV blocking agent, and an organic/mineral composite UV blocking agent.

The UV blocking agent may, specifically, be at least one selected from the group consisting of titanium dioxide ($TiO_2$), zinc oxide (ZnO), drometrizole, drometrizole trisiloxane, digalloyl trioleate, disodium phenyl dibenzimidazole tetrasulfonate, diethylamino hydroxybenzoyl hexyl benzoate, diethylhexyl butamido triazone, methylene bis-benzotriazolyl tetramethylbutylphenol, methyl anthranilate, benzophenone-4, benzophenone-8, bis-ethylhexyloxyphenol methoxyphenyl triazine, ethylhexyl dimethyl PABA, ethylhexyl salicylate, ethylhexyl triazone, isoamyl p-methoxycinnamate, terephthalylidene dicamphor sulfonic acid, phenylbenzimidazole sulfonic acid, and polysilicone-15.

In an embodiment, the UV blocking agent may be at least one selected from titanium dioxide ($TiO_2$) and zinc oxide (ZnO).

The content of the UV blocking agent may be 0.1% by weight or more, 1% by weight or more, 3% by weight or more, 5% by weight or more, 10% by weight or more, or 20% by weight or more, and 50% by weight or less, 40% by weight or less, 30% by weight or less, or 25% by weight or less, based on the total weight of the cosmetic composition.

As a specific example, the content of the UV blocking agent may be 5% by weight to 35% by weight based on the total weight of the cosmetic composition.

In addition, the cosmetic composition may comprise the UV blocking agent in an amount of 1 to 6 parts by weight, 1.5 to 6 parts by weight, or 2 to 4 parts by weight, relative to 1 part by weight of the copolymeric polyhydroxyalkanoate (PHA).

The cosmetic composition according to the present disclosure may comprise an oil. The oil functions as a moisturizer to protect the skin and also functions as a softener to soften the cosmetic composition, thereby improving spreadability.

The oil may be, for example, at least one selected from the group consisting of natural oils, vegetable oils, waxes, hydrocarbons, higher fatty acids, higher alcohols, and silicone oils.

In an embodiment, the oil may be at least one selected from the group consisting of dimethicone, cyclomethicone, cyclopentasiloxane, neopentyl glycol dicaprate, ethylhexyl palmitate, C12-15 alkyl benzoate, dicaprylyl carbonate, hexyl laurate, caprylic/capric triglyceride, propylene glycol dicaprylate/dicaprate, and coco-caprylate/caprate.

The content of the oil may be 0.1% by weight or more, 1% by weight or more, 3% by weight or more, 5% by weight or more, 10% by weight or more, or 15% by weight or more, and 70% by weight or less, 50% by weight or less, 30% by weight or less, or 20% by weight or less, based on the total weight of the cosmetic composition, but it is not limited thereto. As a specific example, the content of the oil may be 0.1% by weight to 70% by weight based on the total weight of the cosmetic composition.

In addition, the cosmetic composition may comprise the oil in an amount of 0.1 to 7 parts by weight, 0.5 to 6 parts by weight, or 1 to 5 parts by weight, relative to 1 part by weight of the copolymeric polyhydroxyalkanoate (PHA).

The cosmetic composition according to the present disclosure may comprise a surfactant. The surfactant allows powdered ingredients or cosmetic compositions to be evenly dispersed in a liquid medium and spread evenly throughout the product. In addition, it also serves to prevent powdered ingredients from separating or precipitating in a liquid medium, thereby maintaining product stability. As described above, surfactants may be used as dispersants, emulsifiers, and detergents depending on their function.

The surfactant may be at least one selected from the group consisting of anionic surfactants, cationic surfactants, amphoteric surfactants, nonionic surfactants, polymeric surfactants, natural surfactants, and silicone surfactants.

In an embodiment, the surfactant may comprise at least one (2 or more, 3 or more, or 4 or more) selected from the group consisting of cetearyl olivate, sorbitan olivate, cetearyl alcohol, glyceryl monostearate, glyceryl stearate, PEG-100 stearate, C14-22 alcohols, C12-20 alkyl glucoside, polyglyceryl-6 polyricinoleate, polyglyceryl-3 polyricinoleate, polyglyceryl-10 myristate, potassium cetyl phosphate, cetyl PEG/PPG-10/1 dimethicone, PEG-10 dimethicone, and stearic acid.

The content of the surfactant may be 0.1% by weight or more, 1% by weight or more, 3% by weight or more, 5% by weight or more, 10% by weight or more, or 15% by weight or more, and 40% by weight or less, 30% by weight or less, 25% by weight or less, or 20% by weight or less, based on the total weight of the cosmetic composition, but it is not limited thereto. As a specific example, the content of the surfactant may be 0.1% by weight to 30% by weight based on the total weight of the cosmetic composition.

In addition, the cosmetic composition may comprise the surfactant in an amount of 0.05 to 3 parts by weight or 0.1 to 1.5 parts by weight, relative to 1 part by weight of the copolymeric polyhydroxyalkanoate (PHA).

The cosmetic composition according to the present disclosure may comprise a polyol. The polyol is an ingredient that secures the formulation stability of the cosmetic composition while it increases skin moisturizing power.

The polyol is not particularly limited, but it may comprise at least one selected from the group consisting of glycerin, 1,3-butylene glycol, dipropylene glycol, propylene glycol, polyethylene glycol, and 1,2-hexanediol. Specifically, the polyol may comprise glycerin, which is economical and has excellent moisturizing power.

The content of the polyol may be 0.1% by weight or more, 1% by weight or more, 3% by weight or more, 5% by weight or more, 10% by weight or more, or 15% by weight or more, and 40% by weight or less, 30% by weight or less, 25% by weight or less, or 20% by weight or less, based on the total weight of the cosmetic composition, but it is not limited thereto. As a specific example, the content of the polyol may be 0.1% by weight to 30% by weight based on the total weight of the cosmetic composition.

In addition, the cosmetic composition may comprise the polyol in an amount of 0.05 to 3 parts by weight, 0.1 to 2 parts by weight, or 0.2 to 1 part by weight, relative to 1 part by weight of the copolymeric polyhydroxyalkanoate (PHA).

The cosmetic composition according to the present disclosure may further comprise purified water to control viscosity and secure formulation stability. The purified water is not particularly limited as long as it is commonly known purified water (e.g., distilled water, deionized water, pure water, or the like).

The cosmetic composition according to the present disclosure may further comprise at least one additive selected from the group consisting of a moisturizer, a softener, a thickener, a preservative, a neutralizer, an acidity regulator, and a fragrance, as needed.

The moisturizer further contained in the cosmetic composition according to the present disclosure is an ingredient capable of supplying moisture to the skin.

The softener further contained in the cosmetic composition according to the present disclosure is an ingredient capable of imparting softness to the skin while soothing the skin. The softener is not particularly limited, but it may comprise at least one selected from the group consisting of natural vegetable oils, ester-based oils, hydrocarbon-based oils, higher alcohols, and silicone oils.

The thickener further contained in the cosmetic composition according to the present disclosure is an ingredient that increases the viscosity of the cosmetic composition. The thickener is not particularly limited, but it may comprise at least one selected from the group consisting of polyquaternium-10, carbomer, sodium chloride, methyl cellulose, carboxymethyl cellulose, carboxymethyl hydroxyguanine, hydroxymethyl cellulose, hydroxyethyl cellulose, sodium acryloyldimethyltaurate/VP crosspolymer, ammonium acryloyldimethyltaurate/VP copolymer, hydroxyethyl acrylate/sodium acryloyldimethyl taurate copolymer, polyacrylate crosspolymer-6, stearic acid, carrageenan, xanthan gum, gellan gum, cellulose gum, and bentonite.

The preservative further contained in the cosmetic composition according to the present disclosure is an ingredient that increases the storage stability of the cosmetic composition. The preservative is not particularly limited, but it may comprise at least one selected from the group consisting of ethanol, phenoxyethanol, ethylhexylglycerin, caprylyl glycol, chlorphenesin, and sodium benzoate.

The neutralizer further contained in the cosmetic composition according to the present disclosure is an ingredient that interacts with the preservative contained in the cosmetic composition to increase the safety of the cosmetic composition during its use. The neutralizer is not particularly limited, but it may comprise at least one selected from the group consisting of potassium hydroxide, sodium hydroxide, calcium hydroxide, triethanolamine, trolamine, and aminomethyl propanol.

The acidity regulator further contained in the cosmetic composition according to the present disclosure is an ingredient that regulates the pH of the cosmetic composition. The acidity regulator is not particularly limited, but it may comprise at least one selected from the group consisting of citric acid, acetic acid, propionic acid, oxalic acid, glycolic acid, malonic acid, lactic acid, succinic acid, tartaric acid, aspartic acid, maleic acid, glutaric acid, glutamic acid, gluconic acid, sorbic acid, benzoic acid, ascorbic acid, and salicylic acid.

The fragrance further contained in the cosmetic composition according to the present disclosure is an ingredient that imparts fragrance to the cosmetic composition.

The content of these additives can be appropriately adjusted within a range that does not impair the properties required for the cosmetic composition.

The cosmetic composition according to the present disclosure may be prepared in various formulations.

For example, the cosmetic composition may have the formulation of a sun stick, sun lotion, sunscreen, sun balm, sun spray, softening toner, nourishing toner, lotion, nourishing cream, massage cream, essence, ampoule, eye cream, makeup base cream, primer, foundation, pact, shampoo, treatment, scalp ampoule, pack, spray, body wash, cleansing foam, cleansing lotion, cleansing oil, or powder.

The cosmetic composition has excellent UV blocking capability. For example, the SPF (sun protection factor) of the cosmetic composition may be 20 or more, 25 or more, 30 or more, 35 or more, 40 or more, or 45 or more, specifically, 20 to 60, 30 to 55, or 30 to 50. In addition, the PA (protection factor of UVA) of the cosmetic composition may be 5 or more, 8 or more, 10 or more, or 15 or more, specifically, 5 to 30, 8 to 25, 10 to 25, or 10 to 20.

As a specific example, the cosmetic composition may have an SPF (sun protection factor) of 30 or more and a PA (protection factor of UVA) of 8 or more.

As the cosmetic composition according to the present disclosure comprises the ingredients described above, it can secure formulation stability while having excellent biodegradability and UV blocking capability. In particular, as the cosmetic composition according to the present disclosure comprises the biodegradable polymer particles described above, it is biodegradable to be environmentally friendly while it causes little irritation to the skin and has excellent usability.

Accordingly, the cosmetic composition according to the present disclosure can be used for cosmetic purposes in various fields.

For example, the cosmetic composition may be used in the manufacture of basic cosmetics, color cosmetics, sunscreen cosmetics, and hair care cosmetics.

Accordingly, the present disclosure provides a cosmetic composition for UV blocking, which comprises biodegradable polymer particles, wherein the biodegradable polymer particles comprise a copolymeric polyhydroxyalkanoate (PHA), the copolymeric polyhydroxyalkanoate (PHA) comprises a repeat unit derived from 4-hydroxybutyrate (4-HB) in an amount of 1% by weight to 20% by weight based on the total weight of the copolymeric polyhydroxyalkanoate (PHA), and the cosmetic composition has an SPF (sun protection factor) of 30 or more and a PA (protection factor of UVA) of 8 or more.

Specific examples of the constitution and content of the biodegradable polymer particles and the copolymeric PHA contained in the cosmetic composition for UV blocking may be as described above.

The SPF (sun protection factor) of the cosmetic composition for UV blocking may be 20 or more, 25 or more, 30 or more, 35 or more, 40 or more, or 45 or more, specifically, 20 to 60, 30 to 55, or 30 to 50. In addition, the PA (protection factor of UVA) of the cosmetic composition may be 5 or more, 8 or more, 10 or more, or 15 or more, specifically, 5 to 30, 8 to 25, 10 to 25, or 10 to 20.

Specifically, the cosmetic composition for UV blocking has excellent UVB and UVA blocking booster effects, which can reduce the amount of UV filters that cause irritation in the formulation and help protect the skin from damage caused by ultraviolet rays. In addition, because the PHA particles are round, they provide a smooth feeling of use and improve spreadability, and they impart a soft focusing effect to the skin, thereby helping smoothen the skin texture. In addition, since PHA has a high melting point, it remains stable without changing its particle or shape even when processed at low or high temperatures. Thus, there is no problem when it is applied to emulsified formulations such as lotions and creams that require a process operated at high temperatures.

In addition, the present disclosure provides a cosmetic composition for skin regeneration, which comprises biodegradable polymer particles, wherein the biodegradable polymer particles comprise a copolymeric polyhydroxyalkanoate (PHA), and the copolymeric polyhydroxyalkanoate (PHA) comprises a repeat unit derived from 4-hydroxybutyrate (4-HB) in an amount of 1% by weight to 20% by weight based on the total weight of the copolymeric polyhydroxyalkanoate (PHA).

Specific examples of the constitution and content of the biodegradable polymer particles and the copolymeric PHA contained in the cosmetic composition for skin regeneration may be as described above.

Hereinafter, the present disclosure will be described in more detail with reference to the following examples. But the following Examples are intended to illustrate the present disclosure, and the scope of the Examples is not limited thereto only.

**Embodiments for Carrying Out the Invention**

**Test Example 1: Preparation of O/W sunscreens and evaluation of usability thereof**

Sunscreens with a mineral UV blocking agent of Examples 1 and 2 and Comparative Examples 1 and 2 were prepared according to the preparation method below with the compositions shown in Table 1 below.

The aqueous phase raw materials were charged to a beaker at room temperature. While they were mixed using a homomixer (Premix, Japan), it was checked that the thickener was dissolved transparently. Thereafter, the oil phase raw materials dissolved by heating to 80°C were slowly added thereto. Upon completion of the addition, they were mixed for 10 minutes to be completely dispersed and homogenized. After the beaker was cooled to 60°C, the mineral blocking agent was slowly added with mixing. They were dispersed and homogenized for 10 minutes. The PHA was slowly added with mixing at 60°C, and they were dispersed and homogenized for 10 minutes. After the beaker was cooled to 45°C, the preservative was added and mixed to complete the preparation. The PHA was used after being ground twice with an atomizer, and its average particle diameter (D50) was about 8 μm.

[Table 1]

| | | Raw material (INCI) | Ex. 1 | Ex. 2 | C. Ex. 1 | C. Ex. 2 |
|---|---|---|---|---|---|---|
| | 1 | Water | to 100 | to 100 | to 100 | to 100 |
| | 2 | Glycerin (polyol) | 5.0 | 5.0 | 5.0 | 5.0 |
| | 3 | 1,2-Hexanediol (polyol) | 2.0 | 2.0 | 2.0 | 2.0 |
| | 4 | Hydroxyethyl acrylate/sodium acryloyldimethyltaurate copolymer (thickener) | 0.5 | 0.5 | 0.5 | 0.5 |
| | 5 | Ammonium acryloyldimethyltaurate/VP copolymer (thickener) | 0.2 | 0.2 | 0.2 | 0.2 |
| | 6 | Cetearyl olivate, sorbitan olivate (surfactant) | 3.0 | 3.0 | 3.0 | 3.0 |
| | 7 | Cetearyl alcohol (surfactant) | 2.0 | 2.0 | 2.0 | 2.0 |
| | 8 | Glyceryl monostearate (surfactant) | 0.5 | 0.5 | 0.5 | 0.5 |
| | 9 | Dimethicone (softener) | 3.0 | 3.0 | 3.0 | 3.0 |
| | 10 | Titanium dioxide (UV blocking agent), aluminum hydroxide (dispersant), stearic acid (surfactant) | 25.0 | 25.0 | 25.0 | 25.0 |
| | 11 | PHA (4HB repeat unit content of 0%) | - | - | 10.0 | - |
| | 12 | PHA (4HB repeat unit content of 4%) | - | 10.0 | - | - |
| | 13 | PHA (4HB repeat unit content of 12%) | 10.0 | - | - | - |
| | 14 | Ethylhexylglycerin (preservative) | 0.5 | 0.5 | 0.5 | 0.5 |
| | | Total (part by weight) | 100 | 100 | 100 | 100 |

The usability and satisfaction of Examples 1 and 2 and Comparative Examples 1 and 2 were evaluated.

The evaluation was conducted by 20 people in total, 10 women and 10 men in their 30s to 40s. 2 g of each of the cosmetic compositions of Examples 1 and 2 and Comparative Examples 1 and 2 was applied to the arm and gently rolled to allow for gentle absorption. The usability and satisfaction were evaluated, and the results are shown in Table 2 below. The usability scores for rolling feeling (spreadability), white cast, and finishing touch were evaluated on a scale of 1.0 (very poor), 2.0 (poor), 3.0 (normal), 4.0 (excellent), and 5.0 (very excellent). Here, the lower degree of the white cast whitening was rated as better. In addition, the user satisfaction was rated from 0% (very dissatisfied) to 100% (very satisfied).

[Table 2]

| | Ex. 1 | Ex. 2 | C. Ex. 1 | C. Ex. 2 |
|---|---|---|---|---|
| Rolling feeling | 4.5 | 3.8 | 3.5 | 3.5 |
| White cast | 4.5 | 4.3 | 4.0 | 2.9 |
| Finishing touch | 4.4 | 4.0 | 3.5 | 3.2 |
| User satisfaction | 95% | 80% | 73% | 63% |

As shown in Table 2 above, the cosmetic composition of Example 1 was evaluated as excellent in all items. Specifically, it was evaluated as having an excellent rolling feeling, less white cast, and a refreshing finishing touch. In addition, the cosmetic composition of Example 2 was also evaluated as excellent overall, although its scores were lower than those of Example 1. Although the cosmetic composition of Comparative Example 1 was evaluated as excellent in terms of the white cast as compared with Comparative Example 2, it received lower scores than those of Examples 1 and 2 in terms of rolling feeling and finishing touch, respectively. The cosmetic composition of Comparative Example 2 was evaluated to be the severe white cast and thus received the lowest score in terms of satisfaction.

**Test Example 2: Preparation of** W/O **sunscreens and evaluation of usability, UV blocking, and hiding rate thereof**

Cosmetic compositions of Examples 3 and 4 and Comparative Examples 3 and 4 were prepared according to the preparation method below with the compositions shown in Table 3 below.

The oil phase raw materials were appropriately dispersed. While they were mixed using a homomixer (Premix, Japan), the aqueous phase raw materials were slowly added thereto over 5 minutes and emulsified. They were completely dispersed and homogenized for 10 minutes. While the fragrance was added thereto, they were mixed to be completely dispersed and homogenized, and the preparation was completed.

The PHA was used after being ground twice with an atomizer, and its average particle diameter (D50) was about 8 μm.

[Table 3]

|  | | Raw material | Ex. 3 | Ex. 4 | C. Ex. 3 | C. Ex. 4 |
|---|---|---|---|---|---|---|
| | 1 | Zinc oxide (UV blocking agent) | 10.0 | 10.0 | 10.0 | 10.0 |
| | 2 | Titanium dioxide (UV blocking agent) | 10.0 | 10.0 | 10.0 | 10.0 |
| | 3 | PHA (4HB repeat unit content of 0%) | - | - | 10.0 | - |
| | 4 | PHA (4HB repeat unit content of 4%) | - | 10.0 | - | - |
| | 5 | PHA (4HB repeat unit content of 12%) | 10.0 | - | - | - |
| | 6 | Polyelyceryl-6 polyricinoleate (surfactant) | 1.0 | 1.0 | 1.0 | 1.0 |
| | 7 | C12-15 akylbenzoate (softener) | 20.0 | 20.0 | 20.0 | 20.0 |
| | 8 | Dicaprylyl carbonate (softener) | 10.0 | 10.0 | 10.0 | 10.0 |
| | 9 | Cetyl PEG/PPG-10/1 dimethicone (surfactant) | 2.5 | 2.5 | 2.5 | 2.5 |
| | 10 | Water | to 100 | to 100 | to 100 | to 100 |
| | 11 | Sodium chloride (thickener) | 1.0 | 1.0 | 1.0 | 1.0 |
| | 12 | 1,2-Hexanediol (polyol) | 2.0 | 2.0 | 2.0 | 2.0 |
| | 13 | Butylene glycol (polyol) | 3.0 | 3.0 | 3.0 | 3.0 |
| | 14 | Fragrance | Appropriate amount | Appropriate amount | Appropriate amount | Appropriate amount |
| | | Total (part by weight) | 100 | 100 | 100 | 100 |

The usability and satisfaction of Examples 3 and 4 and Comparative Examples 3 and 4 were evaluated.

The evaluation was conducted by 20 people in total, 10 women and 10 men in their 30s to 40s. 2 g of each of the cosmetic compositions of Examples 3 and 4 and Comparative Examples 3 and 4 was applied to the arm and gently rolled to allow for gentle absorption. The usability and satisfaction were evaluated in the same manner as in Test Example 1, and the results are shown in Table 4 below.

[Table 4]

|  | Ex. 3 | Ex. 4 | C. Ex. 3 | C. Ex. 4 |
|---|---|---|---|---|
| Rolling feeling | 4.5 | 4.0 | 3.8 | 3.4 |

(continued)

|  | Ex. 3 | Ex. 4 | C. Ex. 3 | C. Ex. 4 |
|---|---|---|---|---|
| White cast | 4.3 | 4.1 | 3.9 | 2.7 |
| Finishing touch | 4.4 | 3.5 | 3.2 | 3.0 |
| User satisfaction | 93% | 81% | 73% | 58% |

As shown in Table 4 above, the cosmetic composition of Example 3 was evaluated as excellent in all items. Specifically, it was evaluated as having an excellent rolling feeling, less white cast, and a soft finishing touch. The cosmetic composition of Example 4 was also evaluated as excellent overall, although its scores were lower than those of Example 3. Although the cosmetic composition of Comparative Example 3 was evaluated as above average in terms of the white cast, it received lower scores than those of Examples 3 and 4 in terms of rolling feeling and finishing touch, respectively. The cosmetic composition of Comparative Example 4 was evaluated to be the severe white cast and thus received the lowest score in terms of satisfaction.

Further, the *in vitro* UV blocking effect of the cosmetic compositions of Examples 1 to 4 and Comparative Examples 1 to 4 was measured using an SPF-290AS (Solar Light, USA) device. The results of the UV blocking test are summarized in Table 5 below.

In addition, since the white cast is an essential factor to be considered when a sunscreen with a mineral composition is manufactured, the hiding index of the cosmetic compositions of Examples 1 to 4 and Comparative Examples 1 to 4 was measured to confirm the white cast. The results are shown in Table 5 below.

The hiding index was calculated using the following Equation 1. The closer it is to 1, the higher the hiding rate (i.e., white cast or covering power). To this end, 1 g of each of the cosmetic compositions of Examples 1 to 4 and Comparative Examples 1 to 4 was applied onto black and white paper using a film-forming applicator to form a film. After drying for 1 hour, the L value was measured using a colorimeter (Coloremeter NR-60CP).

[Equation 1]

$$\text{Hiding index} = \text{L value of film on white paper} / \text{L value of film on black paper}$$

[Table 5]

|  | Ex. 1 | Ex. 2 | C. Ex. 1 | C. Ex. 2 | Ex. 3 | Ex. 4 | C. Ex. 3 | C. Ex. 4 |
|---|---|---|---|---|---|---|---|---|
| SPF (UVB) | 35.72 | 31.56 | 25.21 | 16.90 | 45.08 | 32.45 | 27.3 | 19.09 |
| PA (UVA) | 12.07 | 9.58 | 8.10 | 5.88 | 16.92 | 9.91 | 8.02 | 7.88 |
| Hiding index | 1.89 | 1.83 | 1.79 | 1.48 | 1.98 | 1.89 | 1.78 | 1.65 |

As shown in Table 5 above, the cosmetic composition of Example 3 containing mineral UV blocking agents, such as zinc oxide and titanium dioxide, and a PHA (4HB repeat unit content of 12%) showed the highest UV blocking rate. In addition, the cosmetic composition of Example 1 containing titanium dioxide, even without zinc oxide, and a PHA (4HB repeat unit content of 12%) also showed a relatively high UV blocking rate. The UV blocking rates of Examples 2 and 4 were also excellent.

In contrast, the cosmetic compositions of Comparative Examples 2 and 4, which did not contain a PHA despite containing a mineral UV blocking agent, showed the lowest UV blocking rates. Meanwhile, the PHA (4HB repeat unit content 0%) in Comparative Examples 1 and 3 was shown to contribute relatively to the UV blocking effect. Thus, it was confirmed that both SPF (UVB) and PA (UVA) were increased when a PHA was employed.

In addition, normally, when titanium dioxide is added in large amounts, the white cast increases. However, Examples 1 and 2 and Comparative Example 1 containing a PHA had higher transparency than Comparative Example 2. The same was true for the cosmetic compositions of Examples 3 and 4 and Comparative Example 3 prepared as W/O creams. From this, it is understood that a PHA helps disperse a mineral UV blocking agent.

When the usability, UV blocking, and hiding rate are comprehensively considered as described above, it is understood that the cosmetic compositions of Examples 1 to 4 produced satisfactory effects.

**Test Example 3: Preparation of makeup bases and evaluation of usability thereof**

Cosmetic compositions of Example 5 and Comparative Examples 5 and 6 were prepared according to the preparation method below with the compositions shown in Table 6 below.

The oil phase raw materials were appropriately dispersed. While they were mixed using a homomixer (Premix, Japan), the aqueous phase raw materials were slowly added thereto over 5 minutes and emulsified. They were completely dispersed and homogenized for 10 minutes. After the fragrance was added thereto, they were mixed to be completely dispersed and homogenized, and the preparation was completed.

The PHA was used after being ground three times with an atomizer, and its average particle diameter (D50) was about 5 $\mu$m.

[Table 6]

| | | Raw material | Ex. 5 | C. Ex. 5 | C. Ex. 6 |
|---|---|---|---|---|---|
| | 1 | Neopentyl glycol dicaprate (softener) | 5.0 | 5.0 | 5.0 |
| | 2 | Polyglyceryl-6 polyricinoleate (surfactant) | 1.0 | 1.0 | 1.0 |
| | 3 | Cyclomethicone (softener) | 15.0 | 15.0 | 15.0 |
| | 4 | Dimethicone (softener) | 2.0 | 2.0 | 2.0 |
| | 5 | Dimethicone/vinyl dimethicone crosspolymer (dispersant, softener) | 4.0 | 4.0 | 4.0 |
| | 6 | Titanium dioxide, chrome green oxide, talc (UV blocking agent, pigment) | 7.0 | 7.0 | 7.0 |
| | 7 | PHA (4HB repeat unit content of 12%) | 3.0 | 0.0 | 0.0 |
| | 8 | Polymethylsilsesquioxane (skin conditioning agent) | 0.0 | 3.0 | 0.0 |
| | 9 | Water | to 100 | to 100 | to 100 |
| | 10 | Sodium chloride (thickener) | 1.0 | 1.0 | 1.0 |
| | 11 | 1,2-Hexanediol (polyol) | 2.0 | 2.0 | 2.0 |
| | 12 | Glycerin (polyol) | 3.0 | 3.0 | 3.0 |
| | 13 | Fragrance | Appropriate amount | Appropriate amount | Appropriate amount |
| | | Total (part by weight) | 100 | 100 | 100 |

The above component 6 was obtained by homogeneously mixing titanium dioxide, chromium green oxide, and talc at a weight ratio of 65:7:28, and grinding the mixture twice with an atomizer.

The usability and sensory properties of Example 5 and Comparative Examples 5 and 6 were evaluated. The evaluation was conducted by 20 women in their 30s to 40s. 2 g of each of the cosmetic compositions of Example 5 and Comparative Examples 5 and 6 was applied to the arm and gently rolled to allow for gentle absorption. The usability and sensory properties were evaluated, and the results are shown in Table 7 below. The scores for rolling feeling (spreadability), adherence, durability, soft focus effect, and makeup finishing touch were evaluated on a scale of 1.0 (very poor), 2.0 (poor), 3.0 (normal), 4.0 (excellent), and 5.0 (very excellent).

[Table 7]

| | Ex. 5 | C. Ex. 5 | C. Ex. 6 |
|---|---|---|---|
| Rolling feeling | 4.5 | 4.2 | 3.6 |
| Adherence | 4.3 | 4.3 | 3.8 |
| Durability | 4.3 | 4.2 | 3.5 |
| Soft focus effect | 4.2 | 4.1 | 3.1 |
| Makeup finishing touch | 4.0 | 3.9 | 3.2 |

As shown in Table 7 above, the cosmetic composition of Example 5 was evaluated as excellent in all items. Specifically, it was evaluated as excellent in rolling feeling, adhesion, durability, soft focus effect, and makeup finishing touch. It is understood from the above that the cosmetic composition containing a PHA improves the heavy feeling of use and thick cosmetic finish of the colorant (clay mineral).

In particular, it is understood that a cosmetic composition containing a PHA provides an excellent feeling of use equivalent to polymethylsilsesquioxane, a general silicone powder used to improve the finishing touch and a soft focus effect of a makeup base.

Test Example 4: Preparation of skin regeneration creams and *in-vitro* cytotoxicity recovery evaluation thereof

Cosmetic compositions of Examples 6 and 7 and Comparative Example 7 were prepared according to the preparation method below with the compositions shown in Table 8 below.

The aqueous phase raw materials were charged to a beaker and heated to 80°C. While they were mixed using a homomixer (Premix, Japan), the oil phase raw materials dissolved by heating to 80°C were slowly added thereto and emulsified. They were completely dispersed and homogenized for 10 minutes. It was cooled to 60°C, and the thickener was added thereto. They were mixed for 5 minutes to be completely dispersed and homogenized. It was cooled to 50°C, and the neutralizer was added thereto. They were mixed for 5 minutes to be completely dispersed and homogenized, and the preparation was completed.

The PHA was used after being ground three times with an atomizer, and its average particle diameter (D50) was about 5 $\mu$m.

[Table 8]

| | | Raw material | Ex. 6 | Ex. 7 | C. Ex. 7 |
|---|---|---|---|---|---|
| | 1 | Water | to 100 | to 100 | to 100 |
| | 2 | Glycerin (polyol) | 8.0 | 8.0 | 8.0 |
| | 3 | Ammonium acryloyldimethyltaurate/VP copolymer (thickener) | 0.3 | 0.3 | 0.3 |
| | 4 | 1,2-Hexanediol (polyol) | 2.0 | 2.0 | 2.0 |
| | 5 | PHA (4HB repeat unit content of 12%) | 5.0 | 2.5 | 0.0 |
| | 6 | Cetearyl olivate, sorbitan olivate (surfactant) | 2.0 | 2.0 | 2.0 |
| | 7 | Glyceryl stearate, PEG-100 stearate (surfactant) | 2.5 | 2.5 | 2.5 |
| | 8 | C14-22 alcohols, C12-20 alkyl glucoside (surfactant) | 1.0 | 1.0 | 1.0 |
| | 9 | Propylene glycol dicaprylate/dicaprate (softener) | 3.5 | 3.5 | 3.5 |
| | 10 | Caprylic/capric triglyceride (softener) | 8.0 | 8.0 | 8.0 |
| | 11 | Carbomer 2% solution (thickener) | 5.0 | 5.0 | 5.0 |
| | 12 | Potassium hydroxide 10% solution (neutralizer) | 1.0 | 1.0 | 1.0 |
| | | Total (part by weight) | 100 | 100 | 100 |

Keratinocytes were seeded at a concentration of $2 \times 10^5$ cells/well in a 24-well plate in DMEM (Dulbeco's Modified Eagle's Medium, Gibco) supplemented with 10% FBS and cultured for 1 day in a humidified 37°C, 5% $CO_2$ incubator. After it was replaced with serum-free DMEM, cells were exposed to fluorescent sunlight in the UV wavelength range of 240 to 360 nm for 1 minute to damage them. After the UV exposure, the cosmetic compositions of Examples 6 and 7 and Comparative Example 7 were each inoculated at a certain concentration (v/v) and cultured for 3 days. After the culture, the cell proliferation effect was measured using MTT analysis, and the results are shown in Table 9 below.

[Table 9]

| Specimen | Concentration (ppm) | Cell proliferation effect (%) (relative to UV treatment) |
|---|---|---|
| Ex. 6 | 10,000 | 113.10 % ± 1.27 |
| Ex. 6 | 1,000 | 104.34 % ± 2.01 |
| Ex. 7 | 10,000 | 107.17 % ± 1.79 |

(continued)

| Specimen | Concentration (ppm) | Cell proliferation effect (%) (relative to UV treatment) |
|---|---|---|
| Ex. 7 | 1,000 | 101.11 % ± 1.25 |
| C. Ex. 7 | 10,000 | - |
| C. Ex. 7 | 1,000 | - |

As shown in Table 9 above, the cell proliferation effect of the specimen treated with 10,000 ppm of the cosmetic composition of Example 6 was evaluated to be the best, followed by the specimen treated with 10,000 ppm of the cosmetic composition of Example 7.

In contrast, in the cosmetic composition of Comparative Example 7, cell proliferation was not achieved, from which it is understood that the cell proliferation effect of Examples 6 and 7 was caused by the proliferation by virtue of the employment of a PHA. Specifically, the specimens treated with the cosmetic compositions containing a PHA showed a good cell proliferation rate. It was confirmed that there was no cytotoxicity as the cell proliferation was observed without any decrease.

Test Example 5: Evaluation of formulation stability

The formulation stability of the cosmetic compositions of Examples 1 to 7 and Comparative Examples 1 to 7 prepared as above was evaluated.

The cosmetic compositions were each evaluated for the long-term storage stability at 4°C, 25°C, 45°C, and 50°C for 1 week, 2 weeks, 1 month, and 3 months. The evaluation criteria therefor are summarized in Table 10 below, and the evaluation results are shown in Table 11.

[Table 10]

| Status maintained | ○ |
|---|---|
| Sedimentation/separation and precipitation | × |
| Discoloration | × |
| Change in fragrance | × |

[Table 11]

| Storage temp. | Storage period | Ex. 1 | Ex. 2 | Ex. 3 | Ex. 4 | Ex. 5 | Ex. 6 | Ex. 7 |
|---|---|---|---|---|---|---|---|---|
| 4°C | 1 week | ○ | ○ | ○ | ○ | ○ | ○ | ○ |
| | 2 weeks | ○ | ○ | ○ | ○ | ○ | ○ | ○ |
| | 1 month | ○ | ○ | ○ | ○ | ○ | ○ | ○ |
| | 3 months | ○ | ○ | ○ | ○ | ○ | ○ | ○ |
| 25°C | 1 weeks | ○ | ○ | ○ | ○ | ○ | ○ | ○ |
| | 2 weeks | ○ | ○ | ○ | ○ | ○ | ○ | ○ |
| | 1 month | ○ | ○ | ○ | ○ | ○ | ○ | ○ |
| | 3 months | ○ | ○ | ○ | ○ | ○ | ○ | ○ |
| 45°C | 1 weeks | ○ | ○ | ○ | ○ | ○ | ○ | ○ |
| | 2 weeks | ○ | ○ | ○ | ○ | ○ | ○ | ○ |
| | 1 month | ○ | ○ | ○ | ○ | ○ | ○ | ○ |
| | 3 months | ○ | ○ | ○ | ○ | ○ | ○ | ○ |

(continued)

| Storage temp. | Storage period | Ex. 1 | Ex. 2 | Ex. 3 | Ex. 4 | Ex. 5 | Ex. 6 | Ex. 7 |
|---|---|---|---|---|---|---|---|---|
| 50°C | 1 weeks | ○ | ○ | ○ | ○ | ○ | ○ | ○ |
| | 2 weeks | ○ | ○ | ○ | ○ | ○ | ○ | ○ |
| | 1 month | ○ | ○ | ○ | ○ | ○ | ○ | ○ |
| | 3 months | ○ | ○ | ○ | ○ | ○ | ○ | ○ |

As shown in Table 11 above, the cosmetic compositions of Examples 1 to 7 overall had excellent long-term storage stability.

However, in the particles with a 4HB content of 0% in the PHA, such as Comparative Examples 1 and 3, it was not easy to prepare uniform particles during grinding due to the high crystallinity and brittle nature. When the cosmetic composition formulations of Comparative Examples 1 and 3 were spread, a foreign substance was sometimes felt from the PHA particles.

Although cosmetic compositions of various formulations were prepared through the examples, the scope of the present disclosure is not limited thereto. It can also be prepared in the form of softening toner, nourishing toner, lotion, nourishing cream, massage cream, essence, eye cream, BB cream, CC cream, primer, foundation, pact, shampoo, treatment, scalp ampoule, cleansing cream, cleansing foam, cleansing water, pack, spray, powder, and the like.

**Claims**

1.  A cosmetic composition, which comprises biodegradable polymer particles, wherein the biodegradable polymer particles comprise a copolymeric polyhydroxyalkanoate (PHA),

    the copolymeric polyhydroxyalkanoate (PHA) comprises a repeat unit derived from 4-hydroxybutyrate (4-HB) in an amount of 1% by weight to 20% by weight based on the total weight of the copolymeric polyhydroxyalkanoate (PHA), and
    wherein the biodegradable polymer particles have an average particle diameter of 20 $\mu$m or less.

2.  The cosmetic composition of claim 1, wherein the copolymeric polyhydroxyalkanoate (PHA) further comprises a repeat unit derived from at least one monomer selected from the group consisting of 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-Hhep), 3-hydroxyoctanoate (3-HO), 3-hydroxynonanoate (3-HN), 3-hydroxydecanoate (3-HD), 3-hydroxydodecanoate (3-HDd), 4-hydroxyvalerate (4-HV), 5-hydroxyvalerate (5-HV), and 6-hydroxyhexanoate (6-HH).

3.  The cosmetic composition of claim 1, wherein the copolymeric polyhydroxyalkanoate (PHA) comprises a poly(3-hydroxybutyrate-co-4-hydroxybutyrate) (P3HB-co-4HB).

4.  The cosmetic composition of claim 1, wherein the copolymeric polyhydroxyalkanoate (PHA) comprises a repeat unit derived from 4-hydroxybutyrate (4-HB) in an amount of 3% by weight to 15% by weight based on the total weight of the copolymeric polyhydroxyalkanoate (PHA).

5.  The cosmetic composition of claim 1, wherein the copolymeric polyhydroxyalkanoate (PHA) is employed in an amount of 0.1% by weight to 30% by weight based on the total weight of the cosmetic composition.

6.  The cosmetic composition of claim 1, wherein the cosmetic composition further comprises at least one selected from the group consisting of a UV blocking agent, an oil, a surfactant, and a polyol.

7.  The cosmetic composition of claim 6, wherein the surfactant is at least one selected from the group consisting of anionic surfactants, cationic surfactants, amphoteric surfactants, nonionic surfactants, polymeric surfactants, natural surfactants, and silicone surfactants.

8.  The cosmetic composition of claim 6, wherein the UV blocking agent is at least one selected from the group consisting of titanium dioxide (TiO$_2$), zinc oxide (ZnO), drometrizole, drometrizole trisiloxane, digalloyl trioleate, disodium phenyl dibenzimidazole tetrasulfonate, diethylamino hydroxybenzoyl hexyl benzoate, diethylhexyl butamido triazone, methylene bis-benzotriazolyl tetramethylbutylphenol, methyl anthranilate, benzophenone-4, benzophenone-8, bis-ethylhexyloxyphenol methoxyphenyl triazine, ethylhexyl dimethyl PABA, ethylhexyl salicylate, ethylhexyl triazone, isoamyl p-methoxycinnamate, terephthalylidene dicamphor sulfonic acid, phenylbenzimidazole sulfonic acid, and polysilicone-15.

9. The cosmetic composition of claim 6, wherein the oil is at least one selected from the group consisting of natural oils, vegetable oils, waxes, hydrocarbons, higher fatty acids, higher alcohols, and silicone oils.

10. The cosmetic composition of claim 6, wherein the content of the surfactant is 0.1% by weight to 30% by weight based on the total weight of the cosmetic composition.

11. The cosmetic composition of claim 6, wherein the content of the UV blocking agent is 5% by weight to 35% by weight based on the total weight of the cosmetic composition.

12. The cosmetic composition of claim 6, wherein the content of the oil is 0.1% by weight to 70% by weight based on the total weight of the cosmetic composition.

13. The cosmetic composition of claim 1, wherein the cosmetic composition has an SPF (sun protection factor) of 30 or more and a PA (protection factor of UVA) of 8 or more.

14. The cosmetic composition of claim 1, wherein the cosmetic composition has the formulation of a sun stick, sun lotion, sunscreen, sun balm, sun spray, softening toner, nourishing toner, lotion, nourishing cream, massage cream, essence, ampoule, eye cream, makeup base cream, primer, foundation, pact, shampoo, treatment, scalp ampoule, pack, spray, body wash, cleansing foam, cleansing lotion, cleansing oil, or powder.

15. The cosmetic composition of claim 1, wherein the cosmetic composition is used in the manufacture of basic cosmetics, color cosmetics, sunscreen cosmetics, or hair care cosmetics.

16. A cosmetic composition for UV blocking, which comprises biodegradable polymer particles,

wherein the biodegradable polymer particles comprise a copolymeric polyhydroxyalkanoate (PHA),
the copolymeric polyhydroxyalkanoate (PHA) comprises a repeat unit derived from 4-hydroxybutyrate (4-HB) in an amount of 1% by weight to 20% by weight based on the total weight of the copolymeric polyhydroxyalkanoate (PHA), and
wherein the cosmetic composition has an SPF (sun protection factor) of 30 or more and a PA (protection factor of UVA) of 8 or more.

17. A cosmetic composition for skin regeneration, which comprises biodegradable polymer particles,

wherein the biodegradable polymer particles comprise a copolymeric polyhydroxyalkanoate (PHA), and
the copolymeric polyhydroxyalkanoate (PHA) comprises a repeat unit derived from 4-hydroxybutyrate (4-HB) in an amount of 1% by weight to 20% by weight based on the total weight of the copolymeric polyhydroxyalkanoate (PHA).

## INTERNATIONAL SEARCH REPORT

| International application No. |
| --- |
| **PCT/KR2024/008235** |

**A.    CLASSIFICATION OF SUBJECT MATTER**

**A61K 8/85**(2006.01)i; **A61K 8/02**(2006.01)i; **A61K 8/29**(2006.01)i; **A61Q 19/00**(2006.01)i; **A61Q 17/04**(2006.01)i

According to International Patent Classification (IPC) or to both national classification and IPC

**B.    FIELDS SEARCHED**

Minimum documentation searched (classification system followed by classification symbols)

A61K 8/85(2006.01); A61K 8/02(2006.01); A61K 8/31(2006.01); A61K 8/9728(2017.01); A61K 8/99(2006.01); A61Q 1/02(2006.01); A61Q 19/10(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 폴리하이드록시알카노에이트(polyhydroxyalkanoate, PHA), 4-하이드록시부티레이트(4-hydroxybutyrate, 4-HB), 입자(particle), 화장료(cosmetics), 자외선 차단제(sunscreen)

**C.    DOCUMENTS CONSIDERED TO BE RELEVANT**

| Category* | Citation of document, with indication, where appropriate, of the relevant passages | Relevant to claim No. |
| --- | --- | --- |
| X | KR 10-2023-0070465 A (GRANT INDUSTRIES, INC.) 23 May 2023 (2023-05-23)<br>See paragraphs [0019], [0026], [0032], [0035] and [0105]; examples 2, 8, 10 and 17; tables 3 and 5; and claims 1, 10, 16 and 28. | 1-17 |
| X | WO 2021-262787 A1 (L'ORÉAL et al.) 30 December 2021 (2021-12-30)<br>See page 3, lines 14-15 and page 5, lines 4-6; example 1; and claims 1 and 3. | 1-7,9,10,12,14,15,17 |
| A | KR 10-2023-0003130 A (L'OREAL) 05 January 2023 (2023-01-05)<br>See entire document. | 1-17 |
| A | KR 10-2020-0007779 A (BIO-ON S.P.A.) 22 January 2020 (2020-01-22)<br>See entire document. | 1-17 |
| A | US 2015-0231042 A1 (THE PROCTER & GAMBLE COMPANY) 20 August 2015 (2015-08-20)<br>See entire document. | 1-17 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **19 September 2024** | **19 September 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**EP 4 729 047 A1**

INTERNATIONAL SEARCH REPORT
Information on patent family members

| International application No. |
|---|
| **PCT/KR2024/008235** |

| Patent document cited in search report | | | Publication date (day/month/year) | Patent family member(s) | | | Publication date (day/month/year) |
|---|---|---|---|---|---|---|---|
| KR | 10-2023-0070465 | A | 23 May 2023 | CA | 3191603 | A1 | 24 March 2022 |
| | | | | CN | 116437895 | A | 14 July 2023 |
| | | | | EP | 4213802 | A1 | 26 July 2023 |
| | | | | JP | 2023-542339 | A | 06 October 2023 |
| | | | | MX | 2023003162 | A | 27 March 2023 |
| | | | | US | 2022-0087928 | A1 | 24 March 2022 |
| | | | | WO | 2022-061213 | A1 | 24 March 2022 |
| WO | 2021-262787 | A1 | 30 December 2021 | BR | 112022026553 | A2 | 02 May 2023 |
| | | | | CN | 115484926 | A | 16 December 2022 |
| | | | | EP | 4110285 | A1 | 04 January 2023 |
| | | | | FR | 3113461 | A1 | 25 February 2022 |
| | | | | FR | 3113461 | B1 | 17 November 2023 |
| | | | | US | 2021-0401722 | A1 | 30 December 2021 |
| KR | 10-2023-0003130 | A | 05 January 2023 | BR | 112022026075 | A2 | 17 January 2023 |
| | | | | CN | 115916149 | A | 04 April 2023 |
| | | | | EP | 4167953 | A1 | 26 April 2023 |
| | | | | FR | 3111557 | A1 | 24 December 2021 |
| | | | | FR | 3111557 | B1 | 25 November 2022 |
| | | | | JP | 2023-528858 | A | 06 July 2023 |
| | | | | US | 2023-0293421 | A1 | 21 September 2023 |
| | | | | WO | 2021-260048 | A1 | 30 December 2021 |
| KR | 10-2020-0007779 | A | 22 January 2020 | BR | 112019020152 | A2 | 22 April 2020 |
| | | | | BR | 112019020152 | B1 | 27 December 2022 |
| | | | | CN | 110753569 | A | 04 February 2020 |
| | | | | CN | 115670943 | A | 03 February 2023 |
| | | | | EP | 3600558 | A1 | 05 February 2020 |
| | | | | EP | 3600558 | B1 | 10 January 2024 |
| | | | | EP | 3600558 | C0 | 10 January 2024 |
| | | | | IT | 201700035338 | A1 | 30 September 2018 |
| | | | | JP | 2020-512365 | A | 23 April 2020 |
| | | | | JP | 2022-137133 | A | 21 September 2022 |
| | | | | MX | 2019011568 | A | 13 February 2020 |
| | | | | RU | 2019133535 | A | 30 April 2021 |
| | | | | RU | 2019133535 | A3 | 21 June 2021 |
| | | | | RU | 2765717 | C2 | 02 February 2022 |
| | | | | US | 2021-0113450 | A1 | 22 April 2021 |
| | | | | US | 2023-0046165 | A1 | 16 February 2023 |
| | | | | WO | 2018-178899 | A1 | 04 October 2018 |
| US | 2015-0231042 | A1 | 20 August 2015 | CA | 2937371 | A1 | 20 August 2015 |
| | | | | CA | 2937371 | C | 19 February 2019 |
| | | | | CN | 106029174 | A | 12 October 2016 |
| | | | | EP | 3107627 | A1 | 28 December 2016 |
| | | | | EP | 3107627 | B1 | 21 October 2020 |
| | | | | JP | 2017-505824 | A | 23 February 2017 |
| | | | | US | 9616002 | B2 | 11 April 2017 |
| | | | | WO | 2015-123049 | A1 | 20 August 2015 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015522065 A